# EUROPEAN PATENT APPLICATION

(11) **EP 3 178 509 A1**
(43) Date of publication of application: **14.06.2017**
(21) Application number: 15830011.1
(22) Date of filing: 03.08.2015
(51) Int. Cl.: A61M 5/34

(54) **MEDICAL INSTRUMENT, INJECTION NEEDLE ASSEMBLY, AND DRUG INJECTION APPARATUS**

(30) Priority: 06.08.2014 JP 2014160433
(71) Applicant: Terumo Kabushiki Kaisha, Tokyo 151-0072 (JP)
(72) Inventor: IWASE Yoichiro, Ashigarakami-gun Kanagawa 259-0151 (JP)
(74) Representative: Casalonga
(86) International application number: PCT/JP2015/071960
(87) International publication number: WO 2016/021552

(57) **Abstract**

The invention provides a medical instrument and an injection needle assembly reliably attached to a drug discharge portion without any breakage of a locking mechanism and also provides a drug injection apparatus including the injection needle assembly. In the injection needle assembly of the invention, a drug discharge portion of a syringe is fitted into a fitting portion by taper-fitting and is fixed to the syringe by a locking mechanism. Further, a restriction portion is provided which restricts an operation in which the drug discharge portion is pushed into the fitting portion while the restriction portion contacts the locking mechanism when the drug discharge portion is inserted into the fitting portion and a first screw portion of the locking mechanism is threaded into a second screw portion of the fitting portion. By the restriction portion, an operation in which the drug discharge portion is pushed into the fitting portion is restricted.

## Description

### Technical Field

The present invention relates to a medical instrument and an injection needle assembly connected and fixed to a drug discharge portion including a locking mechanism and also relates to a drug injection apparatus including a locking mechanism.

### Background Art

In a drug injection apparatus, a drug container such as a syringe storing an injection drug and an injection needle assembly including an injectable needle are formed as separate members. The injection needle assembly is used for an injection operation while being attached to a luer portion of a cylinder end of the drug container such as the syringe.

In general, the injection needle assembly is attached to a drug discharge portion (the luer portion) of the syringe by taper-fitting. Specifically, the drug discharge portion is formed in a male tapered shape (a truncated conical shape) in which an outer diameter continuously decreases toward a distal end. The injection needle assembly is provided with a fitting portion having a cylinder hole, the fitting portion being formed in a size corresponding to the drug discharge portion and being formed in a female tapered shape (a truncated conical hole) in which an inner diameter continuously decreases from an opening end of the cylinder hole. The male tapered shape and the female tapered shape are formed to have the same taper ratio. When the drug discharge portion is inserted and pushed into the fitting portion of the injection needle assembly, the tapered portions are fixed in a liquid-tight manner with the faces thereof contacting each other.

In a drug injection apparatus fixed by taper-fitting, various structures with a locking mechanism (a luer locking adapter) have been proposed (see Patent Literature 1). Patent Literature 1 discloses a structure in which a locking mechanism having a female screw is attached to a drug discharge portion (a luer) provided at a distal end of a syringe (an outer cylinder) and a male screw provided in a cap is threaded into the female screw of the locking mechanism so that the syringe and the cap are fixed to each other.

The technology of the locking mechanism is not limited to the drug injection apparatus in which the injection needle assembly or the cap is attached to the syringe. It might also be used in a connection portion of a medical instrument used for a drug injection operation and used while a target connection member such as a medical tube is connected to the syringe.

### Citation List

### Patent Literature

Patent Literature 1: JP 2009-240684 A

### Summary of Invention

### Technical Problem

Incidentally, in the medical instrument of the drug injection apparatus that is fixed by taper-fitting, a problem can arise in which the locking mechanism is separated from the drug discharge portion because the locking mechanism is broken when a user pushes the drug discharge portion into the fitting portion by a strong force. When the locking mechanism is separated from the drug discharge portion, the medical instrument such as the injection needle assembly cannot be reliably attached to the drug discharge portion.

Here, an object of the invention is to provide a medical instrument and an injection needle assembly that can be reliably attached to a drug discharge portion without a breakage of a locking mechanism and to provide a drug injection apparatus including the injection needle assembly.

### Solution to Problem

In order to solve the above-described problems, an injection needle assembly of the invention is an injection needle assembly connected to a drug container including a cylindrical drug discharge portion formed in a male tapered shape so that an outer diameter decreases as it goes toward a distal end and a locking mechanism having a first screw portion and fixed to the vicinity of the drug discharge portion, the injection needle assembly including: a hollow needle which includes a needle tip for puncturing skin; a holding portion which holds the hollow needle; a fitting portion which is formed in a female tapered shape so that the drug discharge portion is inserted thereinto and has a second screw portion formed on an outer peripheral face to be threaded into the first screw portion; and a restriction portion which restricts an operation in which the drug discharge portion is pushed into the fitting portion while the restriction portion contacts the locking mechanism when the drug discharge portion is inserted into the fitting portion and the first screw portion is threaded into the second screw portion.

A drug injection apparatus of the invention includes: a syringe which includes a cylindrical drug discharge portion formed in a male tapered shape so that an outer diameter decreases as it goes toward a distal end and a locking mechanism having a first screw portion and fixed to the vicinity of the drug discharge portion; and an injection needle assembly which is connected to the syringe including a hollow needle, a holding portion holding the hollow needle, a fitting portion which is formed in a female tapered shape so that the drug discharge portion is inserted thereinto and has a second screw portion formed on an outer peripheral face to be threaded into the first screw portion, and a restriction portion which restricts an operation in which the drug discharge portion is pushed into the fitting portion while the restriction portion contacts the locking mechanism when the drug discharge portion is inserted into the fitting portion and the first screw portion is threaded into the second screw portion.

A medical instrument of the invention is a medical instrument connected to a drug container including a cylindrical drug discharge portion formed in a male tapered shape so that an outer diameter decreases as it goes toward a distal end and a locking mechanism having a first screw portion and fixed to the vicinity of the drug discharge portion, the medical instrument including: a fitting portion which is formed in a female tapered shape so that the drug discharge portion is inserted thereinto and has a second screw portion formed on an outer peripheral face to be threaded into the first screw portion; and a restriction portion which restricts an operation in which the drug discharge portion is pushed into the fitting portion while the restriction portion contacts the locking mechanism when the drug discharge portion is inserted into the fitting portion and the first screw portion is threaded into the second screw portion.

### Advantageous Effects of Invention

According to the invention, it is possible to reliably attach a drug discharge portion to a medical instrument such as an injection needle assembly without breaking a locking mechanism.

### Brief Description of Drawings

Fig. 1 is an exploded side view of a drug injection apparatus according to a first embodiment of the invention.
Fig. 2 is an enlarged cross-sectional view of a main part of the drug injection apparatus according to the first embodiment of the invention.
Fig. 3 is an enlarged cross-sectional view of a fitting portion of a first member.
Fig. 4 is a diagram illustrating a (first) case of assembling a drug injection apparatus by fitting a drug discharge portion of a syringe to an injection needle assembly.
Fig. 5 is a (second) diagram illustrating a case of assembling a drug injection apparatus by fitting a drug discharge portion of a syringe to an injection needle assembly.
Fig. 6 is a schematic cross-sectional view of a drug injection apparatus according to a comparative example.
Fig. 7 is a diagram illustrating a measurement result of a force of pushing a drug discharge portion into a fitting portion and a distance of the drug discharge portion inserted into the fitting portion in the drug injection apparatus of the comparative example.
Fig. 8 is a cross-sectional configuration diagram of a drug injection apparatus according to a second embodiment of the invention.
Fig. 9 is a schematic cross-sectional configuration diagram illustrating a case where the drug injection apparatus according to the second embodiment of the invention is assembled.

### Description of Embodiments

Hereinafter, examples of an injection needle assembly and a drug injection apparatus according to an embodiment of the invention will be described with reference to the drawings. Additionally, the invention is not limited to the following examples. A description in the present specification will be made according to the following sequence.

### 1. First Embodiment

### 1-1. Configurations of Injection Needle Assembly and Drug Injection Apparatus

### 1-2. Drug Injection Apparatus Assembling Method

### 2. Second Embodiment

### <<1. First Embodiment>>

### <1-1. Configurations of Injection Needle Assembly and Drug Injection Apparatus>

### [Drug Injection Apparatus]

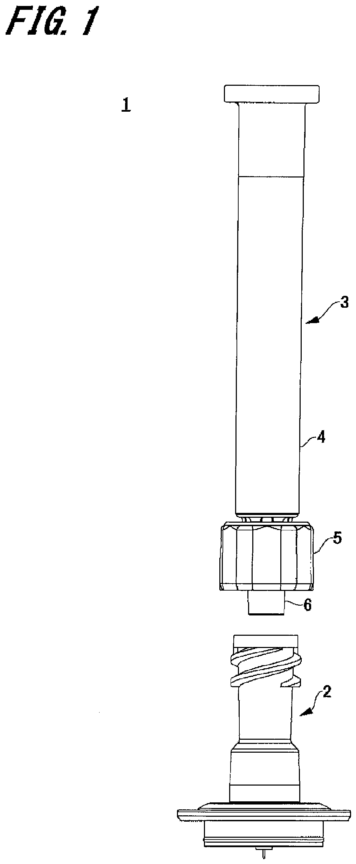

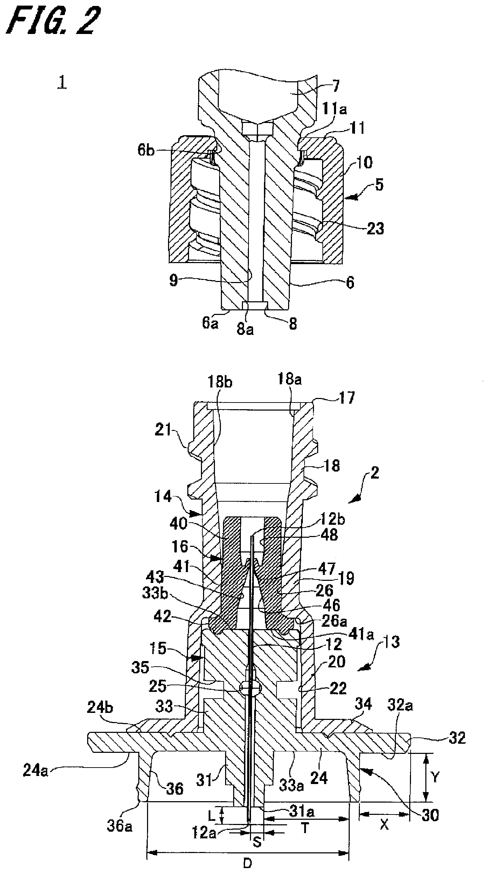

Fig. 1 is an exploded side view of a drug injection apparatus according to a first embodiment of the invention and Fig. 2 is an enlarged cross-sectional view of a main part of the drug injection apparatus illustrated in Fig. 1. A drug injection apparatus 1 is used to puncture a surface of a skin by a needle tip and to inject a drug thereinto. In the embodiment, the drug injection apparatus 1 which is used for a skin injection operation of injecting a drug into an upper skin layer part is illustrated as an example.

The skin includes three parts, that is, an epidermis, a dermis, and a subcutaneous tissue. The epidermis is a layer having a thickness of about 50 to 200 µm from the skin surface and the dermis is a layer having a thickness of about 1.5 to 3.5 mm from the epidermis. Since an influenza vaccine is generally injected into the skin or a muscle, the vaccine is injected into a lower skin layer part or a deeper part therefrom.

A method of decreasing a vaccine injection amount by injecting an influenza vaccine into an upper skin layer part having many immunocompetent cells and corresponding to a target part has been examined. Additionally, the upper skin layer part indicates an epidermis and a dermis in a skin. The drug injection apparatus 1 of the embodiment is the drug injection apparatus 1 used for the skin injection operation in which such an upper skin layer part corresponds to a target part.

The drug injection apparatus 1 of the embodiment includes, as illustrated in Fig. 1, an injection needle assembly 2 and a syringe 3 to which the injection needle assembly 2 is separably attached. Although not illustrated in the drawings, a pushing piece of pushing a drug is inserted into the syringe 3. Further, a locking mechanism 5 is provided at an end of the syringe 3 connected to the injection needle assembly 2.

### [Syringe]

The syringe 3 includes a syringe body 4 and a drug discharge portion 6 which is continuous to the syringe body 4. The syringe body 4 is formed as a cylindrical member and as illustrated in Fig. 2, an inner cylinder portion is formed as a drug storage portion 7 which stores a drug therein. The drug discharge portion 6 is formed as a cylindrical member that is provided at one end of the syringe body 4 in the axial direction and is connected to the drug storage portion 7.

The drug discharge portion 6 is formed in a male tapered shape of which an outer diameter is smaller than the syringe body 4 and decreases as it goes toward a distal end. Here, a taper ratio is expressed as a percentage like A% or a fraction like A/100, for example, when a diameter per 100 mm decreases by A mm. In the embodiment, the male tapered shape forming the drug discharge portion 6 is formed as N/100. Specifically, a luer taper of N = 6 is formed according to IS0594-1 or ISO594-2.

Further, as illustrated in Fig. 2, an extension portion 8 of which a diameter of a cylinder hole 9 increases is provided at a distal end side of the cylinder hole 9 of the drug discharge portion 6. A depth of the extension portion 8, that is, a length from a distal end 6a of the drug discharge portion 6 to an end face 8a of the extension portion 8 in the axial direction is set to about 0.1 mm to 0. 5 mm. Since a diameter of the cylinder hole 9 of the drug discharge portion 6 is formed in a small size in order to suppress a dead volume of a drug as minimal as possible, a distance between a rear end 12b of a hollow needle 12 to be described later and the cylinder hole 9 of the drug discharge portion 6 is narrowed when the syringe 3 and the injection needle assembly 2 are assembled. Then, there is a concern that the rear end 12b of the hollow needle 12 may damage the cylinder hole 9 of the drug discharge portion 6. In the embodiment, since the extension portion 8 is formed, it is possible to prevent a problem in which the rear end 12b of the hollow needle 12 to be described later collides with an inner peripheral face of the cylinder hole 9 of the drug discharge portion 6 so that the cylinder hole 9 is damaged when the syringe 3 and the injection needle assembly 2 are connected to each other to assemble the drug injection apparatus 1.

The distal end 6a of the drug discharge portion 6 is provided with a flat face which is orthogonal to the axial direction thereof and an outer shape of the flat face, that is, an outer edge of a distal end of the drug discharge portion 6 is formed as a circular shape. The flat face of the distal end of the drug discharge portion 6 and the side face (the tapered side face) of the drug discharge portion 6 are connected at the outer edge of the distal end of the drug discharge portion 6. The flat face of the distal end of the drug discharge portion 6 liquid-tightly comes into contact with an end face of an elastic member 16 of the injection needle assembly 2 when the drug discharge portion is attached to the injection needle assembly 2. Further, an outer peripheral face of an end (hereinafter, a rear end) near the syringe body 4 of the drug discharge portion 6 is provided with a concave portion 6b into which a fitting claw 11a of the locking mechanism 5 to be described later is fitted.

The syringe body 4 and the drug discharge portion 6 are formed of synthetic resin (plastic) as an example. As a material of the synthetic resin, polycarbonate, polypropylene, polyethylene, cycloolefin polymer, and the like can be exemplified.

### [Locking Mechanism]

The locking mechanism 5 is provided to cover the outer peripheral face of the drug discharge portion 6 and includes a locking body 10 which is fixed to the injection needle assembly 2 to be described later and a locking mechanism fixing portion 11 which attaches the locking body 10 to the drug discharge portion 6.

The locking body 10 is formed as a cylindrical member that coaxially surrounds the drug discharge portion 6 and the locking body 10 is formed such that an inner periphery is formed in a circular shape and an outer periphery is formed in a hexagonal shape as an example in order for a user to rotate the outer periphery by a hand. The inner peripheral face of the locking body 10 is provided with a female screw portion 23 which is a first screw portion. The female screw portion 23 is formed to be threaded into a male screw portion (a second screw portion) 21 formed at a fitting portion 18 of the injection needle assembly 2 to be described later. In the embodiment, the female screw portion 23 is formed as a double spiral screw groove.

The locking mechanism fixing portion 11 is integrally formed with the locking body 10 at an end near the syringe body 4 of the locking body 10. The locking mechanism fixing portion 11 is formed as a hollow disk-shaped member so that an inner diameter of the locking body 10 decreases and an inner peripheral face of the locking mechanism fixing portion 11 facing the drug discharge portion 6 is provided with the fitting claw 11a which is fitted to the drug discharge portion 6. An inner diameter of the locking mechanism fixing portion 11 is slightly larger than an outer diameter of the drug discharge portion 6. The fitting claw 11a is provided to protrude in the radial direction from the inner peripheral face of the locking mechanism fixing portion 11 facing the drug discharge portion 6 and a plurality of the fitting claws 11a are provided at the same interval in the inner peripheral face of the locking mechanism fixing portion 11.

The lockingmechanism 5 with such a configuration is formed as a member separated from the syringe 3. Then, when the fitting claw 11a is fitted into the concave portion 6b provided at an outer peripheral face of the rear end of the drug discharge portion 6, the movement of the locking mechanism 5 with respect to the drug discharge portion 6 in both the axial direction and the rotation direction is restricted. The locking mechanism 5 can be formed of the same material as those of the syringe body 4 and the drug discharge portion 6.

### [Injection Needle Assembly]

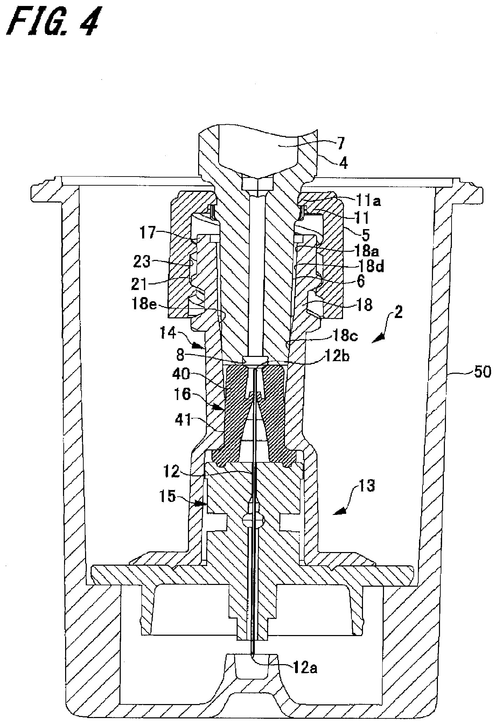

The injection needle assembly 2 includes the hollow needle 12 and a needle hub 13 which holds the hollow needle 12. Further, the injection needle assembly 2 is separably attached to a cap 50 as illustrated in Fig. 4 before the syringe 3 is connected to the injection needle assembly 2.

As a standard (ISO9626:1991/Amd.1:2001(E)) of a medical hollow needle of ISO, the hollow needle 12 having a gauge size of 26 to 33 (the outer diameter of 0.2 to 0.45 mm) is used and the gauge is desirably 30 to 33. Further, it is desirable that an outer diameter be 0.12 to 0.45 mm and an inner diameter be 0.07 to 0.4 mm.

A distal end of the hollow needle 12 is provided with a needle tip 12a having an edge face. Hereinafter, the other end of the hollow needle 12 which is opposite to the needle tip 12a will be referred to as the "rear end 12b". An axial length (hereinafter, referred to as a "bevel length") of the hollow needle 12 in the edge face may be equal to or smaller than 1.4 mm (adult) which is a thinnest thickness of the upper skin layer part to be described later and may be equal to or larger than about 0.5 mm which is the bevel length when a hollow needle having a gauge size of 33 is formed with a single bevel. That is, it is desirable that the bevel length be in the range of 0.5 to 1.4 mm.

Additionally, the bevel length may be equal to or smaller than 0.9 mm (child) which is a thinnest thickness of the upper skin layer part, that is, a bevel length B may be in the range of 0.5 to 0.9 mm. Further, the single bevel indicates an edge face which is generally used in an injection needle and forms 18 to 25° with respect to the longitudinal direction of the needle.

As a material of the hollow needle 12, for example, stainless steel can be exemplified, but the invention is not limited thereto. Other metals such as aluminum, aluminum alloy, titanium, and titanium alloy can be used. Further, the hollow needle 12 may be formed as a straight needle or a tapered needle which is at least partially tapered. As the tapered needle, a structure may be formed such that a base end of the needle has a diameter larger than that of a distal end and an intermediate portion is tapered. Further, a cross-sectional shape of the hollow needle 12 may be a polygonal shape such as a triangular shape other than a circular shape.

The needle hub 13 includes a first member 14 that is provided with the fitting portion 18 into which the drug discharge portion 6 of the syringe 3 is fitted, a second member 15 (a holding portion of the invention) that holds the hollow needle 12, and an elastic member 16 which is sandwiched between the drug discharge portion 6 and the secondmember 15 during the assembly of the druginj ection apparatus 1. In the embodiment, the first member 14 and the second member 15 which constitute the needle hub 13 are formed as separate members, but may be integrally formed with each other.

### [First Member]

The first member 14 is formed in a substantially cylindrical shape on the whole. The first member 14 is provided with a restriction portion 17 which restricts a force applied to the fitting claw 11a while the female screw portion 23 is threaded into the male screw portion 21 when the drug discharge portion 6 is inserted into the fitting portion 18 to contact the locking mechanism 5, the fitting portion 18 into which the drug discharge portion 6 is fitted, an intermediate portion 19 into which the elastic member 16 is inserted, and an insertion portion 20 into which the second member 15 is inserted in order in which the drug discharge portion 6 is fitted.

A cylinder hole 18b of the fitting portion 18 is set to a size corresponding to the drug discharge portion 6 of the syringe 3 and an opening end at one end side is formed as a fitting hole 18a which is fitted to the drug discharge portion 6. Further, a diameter of the fitting portion 18 continuously decreases as it goes from the end of the fitting hole 18a toward the insertion portion 20.

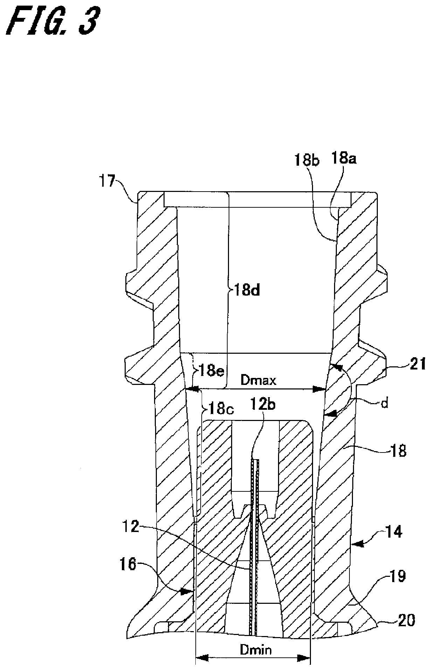

Fig. 3 is an enlarged cross-sectional view of the fitting portion 18 of the first member 14. Referring to Fig. 3, the configurations of the fitting portion 18 and the restriction portion 17 will be further described. The fitting portion 18 includes a fitting wall 18c which is formed at an inner wall of the cylinder hole 18b so as to be fitted to the drug discharge portion 6. As illustrated in Fig. 3, it is desirable that the fitting portion 18 includes a guide wall 18d which is formed at the inner wall of the cylinder hole 18b near the fitting hole 18a. The fitting wall 18c is formed in a female tapered shape in which an inner diameter of the cylinder hole 18b continuously decreases as it goes from the fitting hole 18a toward the insertion portion 20. The tapered shape is formed at a taper ratio of M > 100 as M > N. M is a positive number. In this example, M is set to a value larger than 6 so as to correspond to a state where the taper ratio of the outer diameter of the drug discharge portion 6 is N/100 (in the embodiment, N = 6).

The fitting wall 18c is formed so that the drug discharge portion 6 of the syringe 3 is fittable into the inner diameter of the cylinder hole 18b. When the diameter of the distal end of the drug discharge portion 6 is indicated by K, a diameter (a maximal diameter) of the fitting wall 18c on a side nearer to the fitting hole 18a is indicated by Dmax, and a diameter (a minimal diameter) of the fitting wall 18c on a side nearer to the insertion portion 20 is indicated by Dmin, the cylinder hole 18b of the fitting wall 18c is formed so that a relation of Dmax > K > Dmin is satisfied. The distal end of the drug discharge portion 6 is fitted to a position somewhat near the insertion portion 20 in relation to a position in which the diameter of the cylinder hole 18b becomes K. A fitting length of the fitting wall 18c (a distance from an end near the fitting hole 18a of the fitting wall 18c to a position where the distal end of the drug discharge portion 6 stops when a connection is completed) is 1.8 to 4.8 mm and is desirably 2.3 to 2.8 mm.

The guide wall 18d is formed in such a manner that the inner diameter of the cylinder hole 18b near the fitting hole 18a is widened. It is desirable to form the guide wall 18d so that the inner diameter of the cylinder hole 18b near the fitting hole 18a is shorter than that of a case where the entire cylinder hole 18b is formed at a taper ratio of M/100 by the fitting wall 18c. It is desirable to form the guide wall 18d in a tapered shape so that the inner diameter of the cylinder hole 18b gradually decreases as it goes from the fittinghole 18a toward the insertion portion 20. When a taper ratio at this time is set to M2, a relation of M2 ≤ N is desirable.

A length of the guide wall 18d in the axial direction is set to a length of about 0.5L to 2L as an example when a length of the fitting wall 18c in the axial direction is indicated by L.

Further, it is desirable not to connect the guide wall 18d and the fitting wall 18c in a step shape in order to prevent a leakage of a drug due to the damage of the distal end of the drug discharge portion 6. That is, as illustrated in Fig. 3, a connection wall 18e which connects an end near the insertion portion 20 of the guide wall 18d and an end near the fitting hole 18a of the fitting wall 18c is formed in a tapered shape or a curved face. In a cross-section, an angle d of the connection wall 18e with respect to the axial direction of the drug discharge portion 6 is desirably an obtuse angle (90° < d < 180°) and is more desirably 120° ≤ d ≤ 180°.

An outer peripheral face of the fitting portion 18 is provided with a second screw portion (hereinafter, the male screw portion 21) into which the female screw portion 23 of the locking mechanism 5 is threaded. In the embodiment, the male screw portion 21 is formed as a double spiral screw thread. Additionally, the male screw portion 21 may be a single spiral screw.

The restriction portion 17 is formed at an end of the fitting hole 18a of the fitting portion 18 and is formed as a cylindrical member having a cylinder hole continuous to the cylinder hole 18b of the fitting portion 18. An outer peripheral face of the restriction portion 17 and an outer peripheral face of the fitting portion 18 are formed as a continuous face and an outer diameter thereof is formed to be substantially the same as an outer diameter of the fitting portion 18 near the fitting hole 18a. Further, a step is formed between an inner peripheral face of the restriction portion 17 and an inner peripheral face of the fitting portion 18 and an inner diameter of the restriction portion 17 is set to be slightly larger than an inner diameter of the fitting hole 18a of the fitting portion 18.

Then, a length of the restriction portion 17 in the axial direction is set so that the restriction portion contacts the locking mechanism fixing portion 11 of the locking mechanism 5 during the assembly of the drug injection apparatus 1. The restriction portion 17 is a portion that cancels a force applied to the fitting claw 11a of the locking mechanism 5 when the male screw portion 21 is threaded into the female screw portion 23 while the injection needle assembly 2 is rotated with respect to the drug discharge portion 6 after the drug discharge portion 6 of the syringe 3 is pushed into the fitting portion 18 of the injection needle assembly 2. A function of the restriction portion 17 will be described later.

Next, returning to Fig. 2, a configuration of the insertion portion 20 will be described. A cylinder hole 22 of the insertion portion 20 is set to a size corresponding to a base portion 33 of the second member 15. The insertion portion 20 is provided with a fixing piece 34 which is connected to a connection piece 24 of the second member 15 to be described later. The fixing piece 34 is formed as an annular flange which is continuous to a distal end of the insertion portion 20 and protrudes outward in the radial direction. A surface 24b of the connection piece 24 provided at the second member 15 is fixed to the fixing piece 34 in a contact state. As a method of fixing the fixing piece 34 and the connection piece 24 to each other, for example, an adhesive, ultrasonic welding, laser welding, a fixing screw, and the like can be exemplified.

The intermediate portion 19 is formed as a cylinder hole 26 which is provided between the insertion portion 20 and the fitting portion 18 and has a diameter smaller than that of the cylinder hole 22 of the insertion portion 20. A step face which is located at a boundary between the cylinder hole 22 forming the insertion portion 20 and the cylinder hole 26 forming the intermediate portion 19 becomes an engagement portion 26a which engages with the elastic member 16. A flange portion 42 which will be described later and is provided in the elastic member 16 engages with the engagement portion 26a.

### [Second Member]

The secondmember 15 is provided with a skin contact portion 30 which faces and/or contacts the skin. The skin contact portion 30 is disposed to cover the periphery of the hollow needle 12 when the hollow needle 12 is attached to the second member 15. The skin contact portion 30 includes the substantially columnar base portion 33, an adjustment portion 31, a stabilization portion 36, and a guide portion 32.

The base portion 33 includes end faces 33a and 33b which are perpendicular to the axial direction. The adjustment portion 31 is formed as a columnar convex portion which is provided at a center part of the end face 33a at one end side of the base portion 33 in the axial direction and protrudes in the axial direction of the base portion 33. An axis of the adjustment portion 31 matches an axis of the base portion 33.

The axes of the base portion 33 and the adjustment portion 31 are provided with a penetration hole 25 into which the hollow needle 12 is inserted. Then, the base portion 33 is provided with an injection hole 35 which injects an adhesive (not illustrated) into the penetration hole 25. The injection hole 35 is opened to an outer peripheral face of the base portion 33. Although not illustrated in Fig. 2, the injection hole communicates with the penetration hole 25 to be substantially orthogonal to the penetration hole 25. That is, the hollow needle 12 is fixed to the base portion 33 by an adhesive injected from the injection hole 35 into the penetration hole 25.

The rear end 12b of the hollow needle 12 protrudes from the end face 33b which is the other end of the base portion 33 in the axial direction. The base portion 33 is inserted from the end face 33b into the first member 14 and the rear end 12b of the hollow needle 12 is inserted through an insertion hole 43 which will be described later and is provided in the elastic member 16. Then, the end face 33b of the base portion 33 contacts an end face 41a which will be described later and is provided in the elastic member 16.

Further, the outer peripheral face of the base portion 33 is provided with the connection piece 24. The connection piece 24 is formed as an annular flange which protrudes outward in the radial direction of the base portion 33 and includes surfaces 24a and 24b which face each other in the axial direction of the base portion 33. The first member 14 is connected to the surface 24b of the connection piece 24. Further, the distal end of the connection piece 24 is formed as the guide portion 32. The guide portion 32 will be described in detail later.

An end face of the adjustment portion 31 is formed as a needle protrusion face 31a from which the needle tip 12a of the hollow needle 12 protrudes. The needle protrusion face 31a is formed as a surface which is orthogonal to the axial direction of the hollow needle 12. The needle protrusion face 31a defines a puncture depth of the hollow needle 12 while contacting the skin surface when the hollow needle 12 punctures the upper skin layer part. That is, a depth in which the hollow needle 12 punctures the upper skin layer part is defined by a length (hereinafter, referred to as a "protrusion length L") of the hollow needle 12 protruding from the needle protrusion face 31a.

A thickness of the upper skin layer part corresponds to a depth to a dermis layer from the skin surface and is substantially in the range of 0.5 to 3.0mm. For that reason, the protrusion length L of the hollow needle 12 can be set in the range of 0.5 to 3.0 mm.

By the way, a vaccine is generally injected into an upper arm. However, it is thought that a peripheral part of a shoulder having a thick skin, that is, a deltoid muscle part is suitable in the case of an injection into an upper skin layer part. A thickness of an upper skin layer part of a deltoid muscle was measured for nineteen children and thirty one adults. This measurement was performed while the upper skin layer part having high ultrasonic reflectivity was visualized by an ultrasonic measurement device (NP60R-UBM, high-resolution echo for small animal, Nepa Gene Co., Ltd.). Additionally, since measurement values were log normally distributed, a range of MEAN±2SD was obtained by a geometric average.

As a result, the thickness of the upper skin layer part at the deltoid muscle of a child was 0.9 to 1.6 mm. Further, the thickness of the upper skin layer part at the deltoid muscle of an adult was 1.4 to 2.6 mm at a far part, was 1.4 to 2.5 mm at a center part, and was 1.5 to 2.5 mm at a near part. From the above-describedmeasurement, it was found that the thickness of the upper skin layer part at the deltoid muscle was 0.9 mm or more for a child and was 1. 4 mm or more for an adult. Regarding the injection at the upper skin layer part of the deltoid muscle, it is desirable to set the protrusion length L of the hollow needle 12 in the range of 0.9 to 1.4 mm.

When the protrusion length L is set in this way, the edge face of the needle tip 12a can be reliably positioned to the upper skin layer part. As a result, the needle hole (the drug discharge hole) opened to the edge face can be located at the upper skin layer part in any position within the edge face. Additionally, since a drug flows under the skin from a gap between a side face of an end of the needle tip 12a and a notched skin if the needle tip 12a deeply punctures the upper skin layer part even when the drug discharge hole is located at the upper skin layer part, it is important to reliably position the edge face to the upper skin layer part.

Additionally, in a case where a drug is administrated to the upper skin layer part, a bevel length of 1.0 mm or less cannot be easily formed in the hollow needle thicker than a gauge size of 26. Thus, it is desirable to use a hollow needle which is thinner than a gauge size of 26 in order to set the protrusion length L of the hollow needle 12 within a desirable range.

It is desirable to form the needle protrusion face 31a so that a distance S from a peripheral edge to a peripheral face of the hollow needle 12 becomes 1. 4 mmor less and becomes desirably in the range of 0.3 to 1.4 mm. The distance S from the peripheral edge of the needle protrusion face 31a to the peripheral face of the hollow needle 12 is set in consideration of a pressure applied to a blister formed when a drug is administrated to the upper skin layer part. That is, the needle protrusion face 31a is set to a size which is sufficiently smaller than the blister formed on the upper skin layer part and does not disturb the formation of the blister. As a result, it is possible to prevent a leakage of a drug administrated while the needle protrusion face 31a is pushed against the skin in the periphery of the hollow needle 12.

The stabilization portion 36 is formed in a cylindrical shape protruding from the surface 24a of the connection piece 24 provided in the base portion 33. The hollow needle 12 and the adjustment portion 31 are disposed at the cylinder hole of the stabilization portion 36. That is, the stabilization portion 36 is formed in a cylindrical shape surrounding the periphery of the adjustment portion 31 into which the hollow needle 12 is inserted and is provided to be separated from the needle tip 12a of the hollow needle 12 in the radial direction.

As illustrated in Fig. 2, the end face 36a of the stabilization portion 36 is located near the rear end 12b of the hollow needle 12 in relation to the needle protrusion face 31a of the adjustment portion 31. When the needle tip 12a of the hollow needle 12 punctures a living body, the needle protrusion face 31a first contacts the skin surface and then contacts the end face 36a of the stabilization portion 36. At this time, since the end face 36a of the stabilization portion 36 contacts the skin, the drug injection apparatus 1 is stabilized and thus the hollow needle 12 can be maintained in a posture which is substantially perpendicular to the skin.

Additionally, even when the end face 36a of the stabilization portion 36 is located in the same plane as the needle protrusion face 31a or is located near the needle tip 12a of the hollow needle 12 in relation to the needle protrusion face 31a, the hollow needle 12 can be maintained in a posture which is substantially perpendicular to the skin. Additionally, if the swelling of the skin caused when the stabilization portion 36 is pushed against the skin is considered, it is desirable to set a distance between the end face 36a of the stabilization portion 36 and the needle protrusion face 31a in the axial direction to 1.3 mm or less.

Further, an inner diameter of the stabilization portion 36 is set to a value which is equal to or larger than the diameter of the blister formed on the skin. Specifically, the inner diameter of the stabilization portion is set so that a distance T from the inner wall face of the stabilization portion 36 to the peripheral edge of the needle protrusion face 31a is in a range of 4 mm to 15 mm. Accordingly, it is possible to prevent a problem in which the formation of the blister is disturbed due to a pressure applied from the inner wall face of the stabilization portion 36 to the blister.

The shortest distance T from the inner wall face of the stabilization portion 36 to the outer peripheral face of the adjustment portion 31 is not particularly limited as long as the distance is 4 mm or more. However, since the outer diameter of the stabilization portion 36 increases when the distance T increases, the entire end face 36a of the stabilization portion 36 cannot easily contact the skin when the hollow needle 12 punctures a slender arm of a child. For that reason, it is desirable to set the distance T to 15 mm to maximum in consideration of the slenderness of the arm of the child.

Further, when the distance S from the peripheral edge of the needle protrusion face 31a to the peripheral face of the hollow needle 12 is 0.3 mm or more, the adjustment portion 31 does not enter the skin. Thus, the inner diameter of the stabilizationportion 36 canbe set to 9 mm or more in consideration of the diameter (about 0.3 mm) of the needle protrusion face 31a and the distance T (4 mm or more) from the inner wall face of the stabilization portion 36 to the peripheral edge of the needle protrusion face 31a.

Additionally, the shape of the stabilization portion 36 is not limited to a cylindrical shape and may be, for example, a rectangular cylindrical shape such as a quadratic prism or a hexagonal prism having a cylinder hole formed at a center thereof.

Further, the cap 50 is separably attached to the stabilization portion 36 (see Fig. 4). The cap 50 covers the needle tip 12a of the hollow needle 12. Accordingly, it is possible to prevent the needle tip 12a from contacting a finger of a user when the needle hub 13 is attached to the syringe 3. Further, since the drug injection apparatus 1 or the injection needle assembly 2 which has been used can be maintained in a safe state at all times, the user can discard the drug injection apparatus 1 or the injection needle assembly 2 which has been used without any worry.

The guide portion 32 is a distal end side portion which is located at the outside in the radial direction of the second member 15 in relation to the stabilization portion 36 of the connection piece 24. The guide portion 32 includes a contact face 32a which contacts the skin. The contact face 32a is a surface that is a part of the surface 24a of the connection piece 24 and is substantially parallel to the end face 36a of the stabilization portion 36. When the stabilization portion 36 is pushed until the contact face 32a of the guide portion 32 contacts the skin, a force in which the stabilization portion 36 and the hollow needle 12 push the skin can be maintained at a predetermined value or more at all times. Accordingly, a part (corresponding to the protrusion length L) which protrudes from the needle protrusion face 31a of the hollow needle 12 reliably punctures the skin.

A distance (hereinafter, referred to as a "guide portion height") Y from the contact face 32a of the guide portion 32 to the end face 36a of the stabilization portion 36 is set so that the skin is punctured while the hollow needle 12 and the stabilization portion 36 are pushed against the skin at an appropriate pressure. Additionally, an appropriate force of pushing the hollow needle 12 and the stabilization portion 36 is, for example, 3 to 20 N. As a result, since a force of pushing the hollow needle 12 and the stabilization portion 36 against the skin is guided to a user by the guide portion 32, the needle tip 12a (the edge face) of the hollow needle 12 can be reliably positioned to the upper skin layer part and the user can have a feeling of security.

The height Y of the guide portion 32 is appropriately set on the basis of the inner diameter of the stabilization portion 36 and a length (hereinafter, referred to as a "guide portion length") X from a distal end face of the guide portion 32 to an outer peripheral face of the stabilization portion 36. For example, when an inner diameter D of the stabilization portion 36 is 12 mm and the guide portion length X is 3.0 mm, the guide portion height Y is set in the range of 2.3 to 6.6 mm.

### [Elastic Member]

Next, the elasticmember 16 will be described. The elastic member 16 is disposed inside the cylinder hole 26 which becomes the intermediate portion 19 of the first member 14 and is interposed between the second member 15 and the syringe 3. The elastic member 16 includes a deformation portion 40, a body portion 41, and a flange portion 42 which is provided at one end of the body portion 41 in the axial direction and these components are integrated with one another.

The body portion 41 is formed in a substantially cylindrical shape and the end face 41a which is opposite to the formation side of the deformation portion 40 contacts the end face 33b of the base portion 33 of the secondmember 15. Further, an outer diameter of the body portion 41 is formed to be slightly larger than an inner diameter on the side of the insertion portion 20 of the fitting portion 18 provided at the first member 14. Accordingly, the elastic member 16 is liquid-tightly held by the intermediate portion 19 by the elasticity thereof.

The flange portion 42 is provided at an outer peripheral face on the side of the end face 41a of the body portion 41 and is formed in an annular shape which protrudes outward in the radial direction from the outer peripheral face of the body portion 41. The flange portion is formed to be larger than an inner diameter of the intermediate portion 19 holding the body portion 41 and to be smaller than an inner diameter of the insertion portion 20. For that reason, one surface of the flange portion 42 contacts the engagement portion 26a formed as a step face between the insertion portion 20 and the intermediate portion 19 of the first member 14 and the other plane contacts the end face 33b of the base portion 33 of the second member 15. The elastic member 16 is fixed to the needle hub 13 in such a manner that the flange portion 42 is sandwiched between the engagement portion 26a of the first member 14 and the base portion 33 of the second member 15.

The deformation portion 40 is formed in a cylindrical member that is provided at the opposite side to the end face 41a contacting the base portion 33 in the body portion 41 and an outer diameter thereof is formed to be equal to or a little smaller than the outer diameter of the body portion 41. A side face of the deformation portion 40 is formed to be continuous to a side face of the body portion 41 and an inner wall face of the deformation portion 40 is formed to be continuous to an inner wall face of the body portion 41. Then, an end face 40a which is opposite to the body portion 41 in the deformation portion 40 becomes a contact face contacting the distal end 6a of the drug discharge portion 6. During the assembly of the drug injection apparatus 1, the deformation portion 40 is pushed into the distal end 6a of the drug discharge portion 6 to be crushed toward the body portion 41.

In a case where the drug injection apparatus 1 of the embodiment is used, the user fits the drug discharge portion 6 into the fitting portion 18 and connects the syringe 3 and the injection needle assembly 2 to each other so that the drug injection apparatus 1 is assembled. Then, a depth or a strength in which the drug discharge portion 6 is inserted into the fitting portion 18 becomes different depending on the user. In contrast, since the deformation portion 40 is deformed to absorb a difference in adhesion degree between the drug discharge portion 6 and the fitting portion 18 in accordance with a difference in insertion depth of the drug discharge portion 6 with respect to the fitting portion 18, there is an effect that a pressure resistance is maintained. Further, since the deformation portion 40 is deformed when the drug discharge portion 6 is fitted into the fitting portion 18, there is an effect that a dead volume formed between the distal end 6a of the drug discharge portion 6 and the rear end 12b of the hollow needle 12 can be decreased to 8 µL or less.

Then, in the embodiment, the elastic member 16 is provided with the insertion hole 43 into which the rear end 12b of the hollow needle 12 protruding from the end face 33b of the base portion 33 in the axial direction of the body portion 41 and the deformation portion 40 is inserted. An inner wall face of the insertion hole 43 is provided with an end face side separation portion 46, a contact face side separation portion 48, and an adhesion portion 47.

The end face side separation portion 46 is formed by the inner wall face of the insertion hole 43 near the body portion 41 and forms an opening of the insertion hole 43 in the end face 41a of the body portion 41. The end face side separation portion 4 6 is separated from an outer peripheral face of the hollow needle 12 and is formed in a tapered shape in which a diameter of the insertion hole 43 continuously increases as it goes toward the end face 41a. Accordingly, the rear end 12b of the hollow needle 12 which protrudes from the end face 33b of the base portion 33 can be easily inserted through the insertion hole 43. Additionally, the shape of the end face side separation portion 46 in the insertion hole 43 is not limited to a tapered shape as long as a shape is formed in which the hollow needle 12 is easily inserted through the insertion hole 43.

The contact face side separation portion 48 is formed by the inner wall face of the insertion hole 43 near the deformation portion 40 and forms an opening of the insertion hole 43 in the end face 40a near the deformation portion 40. The contact face side separation portion 48 is separated from the outer peripheral face of the hollow needle 12. Further, an inner wall face forming the contact face side separation portion 48 is formed to be substantially parallel to the axial direction of the deformation portion 40. Since the elastic member 16 is provided with the contact face side separation portion 48 , it is possible to prevent a problem in which the deformation portion 40 covers the rear end 12b of the hollow needle 12 to block the needle hole when the deformation portion 40 is elastically deformed.

The inner wall face which forms the contact face side separation portion 48 in the insertion hole 43 is formed to be substantially parallel to the axial direction of the deformation portion 40, but may be formed in a tapered shape in which the diameter of the insertion hole 43 continuously increases as it goes from the adhesion portion 47 toward an end face of the deformation portion 40. That is, a shape of the contact face side separation portion 48 in the insertion hole 43 may be formed in a shape in which the deformation portion 40 hardly blocks the needle hole of the hollow needle 12 when the deformation portion 40 is elastically deformed.

The adhesion portion 47 is formed between the end face side separation portion 46 and the contact face side separation portion 48 and is formed in the vicinity of a boundary between the body portion 41 and the deformation portion 40. The adhesion portion 47 liquid-tightly adheres to the outer peripheral face of the hollow needle 12. Accordingly, a drug inside the syringe 3 does not intrude into the second member 15 of the needle hub 13 from a gap between the hollow needle 12 and the elastic member 16.

A protrusion length of the hollow needle 12 from the adhesion portion 47 toward the contact face side separation portion 48 is set to a length in which the rear end 12b of the hollow needle 12 is exposed to the cylinder hole 9 of the drug discharge portion 6 when the deformation portion 40 of the elastic member 16 is crushed toward the distal end 6a of the drug discharge portion 6. Since such an elastic member 16 is provided, the distal end 6a of the drug discharge portion 6 crushes the deformation portion 40 of the elastic member 16 so that the distal end 6a of the drug discharge portion 6 liquid-tightly contacts the end face 41a of the deformation portion 40 when the syringe 3 and the injection needle assembly 2 are connected to each other. Then, since the hollow needle 12 is exposed toward the drug discharge portion 6 from the end face 41a of the crushed deformation portion 40, the cylinder hole 9 of the drug discharge portion 6 communicates with the hollow needle 12.

As a material of the elastic member 16, elastic materials including various rubber materials such as natural rubber and silicone rubber, various thermoplastic elastomers such as polyurethane and styrene, or a mixture of these are exemplified.

The injection needle assembly 2 with the above-described configuration is manufactured as below. First, the hollow needle 12 intrudes into the penetration hole 25 of the base portion 33 of the second member 15. In a state where the needle tip 12a of the hollow needle 12 protrudes from the adjustment portion 31 by a length in which the skin is punctured, an adhesive flows from the injection hole 35 formed in the base portion 33 so that the second member 15 and the hollow needle 12 adhere to each other to become a fixed state. Next, the elastic member 16 is inserted into the intermediate portion 19 of the first member 14. Subsequently, an adhesive is applied to the guide portion 32 of the second member 15, the base portion 33 of the second member 15 is inserted into the insertion portion 20 of the first member 14, and the fixing piece 34 of the first member 14 adheres to the guide portion 32 of the second member 15 to become a fixed state. Accordingly, the injection needle assembly 2 is completed.

### <1-2. Drug Injection Apparatus Assembling Method>

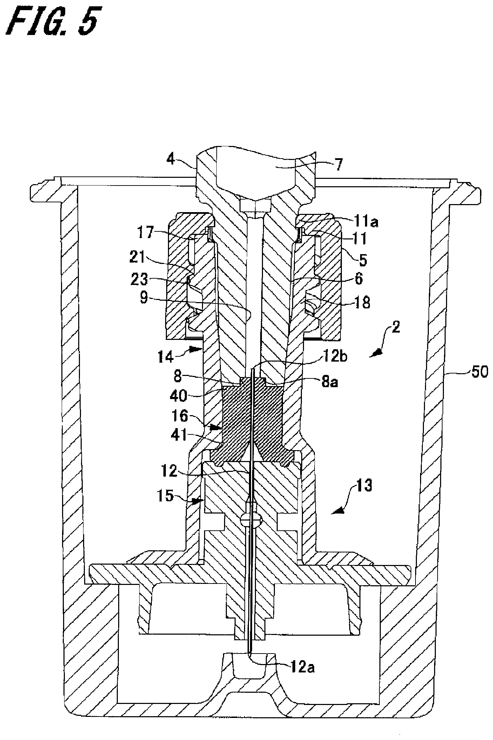

Next, a method of assembling the drug injection apparatus 1 by connecting the injection needle assembly 2 to the drug discharge portion 6 will be described. Figs. 4 and 5 illustrate a case of assembling the drug injection apparatus 1 by fitting the drug discharge portion 6 of the syringe 3 to the injection needle assembly 2.

As illustrated in Fig. 4, the drug discharge portion 6 of the syringe 3 is inserted into the fitting portion 18 of the injection needle assembly in a state where the injection needle assembly 2 is stored in the cap 50. The distal end of the drug discharge portion 6 is inserted into the cylinder hole 18b from the fitting hole 18a of the fitting portion 18. At this time, since an inner diameter of the guide wall 18d is formed to be larger than an outer diameter of the distal end 6a of the drug discharge portion 6, the distal end 6a of the drug discharge portion 6 can be easily inserted into the fitting hole 18a.

The drug discharge portion 6 is further inserted into the cylinder hole 18b. At this time, even when the drug discharge portion 6 is inclined so that the distal end 6a of the drug discharge portion 6 contacts the connection wall 18e, the drug discharge portion 6 smoothly slides on the inner wall of the cylinder hole 18b since the connection wall 18e is obliquely formed in a tapered shape. For this reason, it is possible to prevent a damage of the distal end 6a of the drug discharge portion 6.

When the drug discharge portion 6 is inserted into the cylinder hole 18b to a position where the female screw portion 23 of the locking mechanism 5 contacts the male screw portion 21 of the fitting portion 18, the injection needle assembly 2 and the locking mechanism 5 are relatively rotated so that the male screw portion 21 is threaded into the female screw portion 23. At this time, since the locking mechanism 5 is fixed to the syringe 3, the user can thread the male screw portion 21 into the female screw portion 23 by rotating the syringe 3. Then, the drug discharge portion 6 is deeply inserted into the cylinder hole 18b of the fitting portion 18 by the fastening of the locking mechanism 5.

In this way, when the drug discharge portion 6 is inserted into the fittingportion 18, the drug discharge portion 6 contacts the fitting wall 18c of the fitting portion 18. Since a taper ratio of the fitting wall 18c is set to be larger than a taper ratio of the drug discharge portion 6, only an outer edge of a distal end of the drug discharge portion 6 contacts the fitting wall 18c.

As illustrated in Fig. 5, when the locking mechanism 5 is further fastened, the drug discharge portion 6 further moves toward the elastic member 16 to be deeply inserted into the cylinder hole while the outer edge of the distal end of the drug discharge portion 6 is pushed against the fitting wall 18c. Then, the movement of the drug discharge portion 6 toward the elastic member 16 stops at a time point at which an end face of the restriction portion 17 of the first member 14 contacts the locking mechanism fixing portion 11 of the locking mechanism 5. That is, since the restriction portion 17 contacts a bottom face of the locking mechanism fixing portion 11, the drug discharge portion 6 is not pushed toward the elastic member 16 anymore. Here, the movement of the drug discharge portion 6 toward the elastic member 16 may not be essentially stopped by a configuration in which the restriction portion 17 contacts a bottom portion of the locking mechanism fixing portion 11. Even before the restriction portion 17 contacts the bottom portion of the locking mechanism fixing portion 11, the sufficient adhering of the distal end of the drug discharge portion 6 can be ensured by the fitting wall 18c and the elastic member 16.

Incidentally, the first member 14 is formed of synthetic resin. For this reason, the fitting wall 18c is deformed as a recess due to its material characteristics when the drug discharge portion 6 is inserted into the fitting portion 18 until the end face of the restriction portion 17 contacts the bottom face of the locking mechanism fixing portion 11. Accordingly, the outer edge of the distal end of the drug discharge portion 6 bits into the fitting wall 18c. Thus, the outer edge of the distal end of the drug discharge portion 6 adheres to the fitting wall 18c so that the drug discharge portion 6 and the fitting wall 18c are liquid-tightly fixed.

Further, when the drug discharge portion 6 is inserted into the fitting portion 18 until the end face of the restriction portion 17 contacts the bottom face of the locking mechanism fixing portion 11, the deformation portion 40 of the elastic member 16 is crushed by the drug discharge portion 6 and intrudes into the extension portion 8 provided in the distal end of the drug discharge portion 6. Accordingly, the end face 8a of the extension portion 8 in the axial direction and the distal end 6a of the drug discharge portion 6 liquid-tightly contact the end face of the deformation portion 40 of the elastic member 16. At this time, since the hollow needle 12 is exposed from the distal end of the crushed deformation portion 40 toward the drug discharge portion 6, the cylinder hole 9 of the drug discharge portion 6 communicates with the hollow needle 12.

Then, since the drug discharge portion 6 is inserted into the fitting portion 18 until the end face of the restriction portion 17 contacts the bottom face of the locking mechanism fixing portion 11 so that the injection needle assembly 2 is locked to the drug discharge portion 6 by the locking mechanism 5, the assembly of the drug injection apparatus 1 is completed. Additionally, the insertion depth of the drug discharge portion 6 with respect to the fitting portion 18 is different depending on the user. However, the distal end 6a of the drug discharge portion 6 liquid-tightly contacts the deformation portion 40 of the elastic member 16 to a degree that the female screw portion 23 of the locking mechanism 5 is threaded into the male screw portion 21 of the fitting portion 18. That is, a distance in which the drug discharge portion 6 is inserted into the fitting portion 18 to a position where the restriction portion 17 contacts the locking mechanism fixing portion 11 is a maximal insertion distance. Here, the locking may be performed at a position where the restriction portion 17 does not contact the locking mechanism fixing portion 11. Further, as illustrated in Fig. 5, the body portion 41 of the elastic member 16 is slightly deformed when the drug discharge portion 6 is inserted into the fitting portion 18 until the restriction portion 17 contacts the locking mechanism fixing portion 11.

In the embodiment, the outer edge of the distal end of the drug discharge portion 6 is formed in a small circular shape and when the drug discharge portion 6 is fitted into the fitting portion 18, the outer edge of the small distal end thereof is pushed against the fitting wall 18c. Additionally, a taper ratio of the male tapered shape of the drug discharge portion 6 is formed to be smaller than a taper ratio of the female tapered shape of the fitting portion 18. As a result, a pressure of pushing the drug discharge portion 6 against the fitting wall 18c entirely concentrates on a narrow area of the fitting wall 18c contacting the outer edge of the distal end of the drug discharge portion 6. That is, since a slight gap is formed between the fitting wall 18c and the side face of the drug discharge portion 6, a force concentrates on the outer edge of the distal end of the drug discharge portion 6.

For this reason, even when a force of pushing the injection needle assembly 2 and the drug discharge portion 6 is a weak force, the fitting wall 18c can be easily deformed so that the outer edge of the distal end of the drug discharge portion 6 is easily bitten into the fitting wall 18c. Accordingly, since the injection needle assembly 2 and the drug discharge portion 6 are properly fitted to each other in a liquid-tight state, a leakage of a liquid can be reliably prevented.

In the drug injection apparatus 1 in which the injection needle assembly 2 and the drug discharge portion 6 have the same taper ratio, since there is a need to apply a pressure so that the fitting wall 18c of the injection needle assembly 2 and the side face of the drug discharge portion 6 rub against each other during the assembly, a friction resistance increases. For this reason, a relatively large force is needed when the injection needle assembly 2 and the syringe 3 are assembled. In contrast, in the embodiment, since the taper ratio of the male tapered shape of the drug discharge portion 6 is formed to be smaller than the taper ratio of the female tapered shape of the fitting portion 18, the injection needle assembly 2 and the drug discharge portion 6 can be reliably fitted to each other even at a weak force.

The material of the first member 14 is not limited to a relatively smooth material such as polypropylene. Even a relatively hard material such as polycarbonate or cycloolefin polymer can be used so that the outer edge of the distal end of the drug discharge portion 6 is sufficiently bitten into the fitting wall 18c.

A value M of the taper ratio of the fitting wall 18c may be appropriately set to a value larger than a value N, but when the value M and the value N is not substantially different, an amount in which the outer edge of the distal end of the drug discharge portion 6 is bitten into the fitting wall 18c decreases. For this reason, M ≥ 1. 5N (in the case of N = 6, M ≥ 9) is desirable and M ≥ 2N (in the case of N = 6, M ≥ 12) is more desirable. When the value M is too larger than the value N, the diameter of the cylinder hole increases. For that reason, M ≤ 10N (in the case of N = 6, M ≤ 30) is desirable, M ≤ 5N (in the case of N = 6, M ≤ 30) is more desirable, and M ≤ 3N (in the case of N = 6, M ≤ 18) is further desirable in consideration of a real range obtained by a relation of an outer diameter or a wall thickness of the fitting portion 18. As an exemplary range, the value M is desirable in the range of M = 1.5N to 10N (in the case of N = 6, M = 9 to 60), is more desirable in the range of M = 2N to 5N (in the case of N = 6, M = 12 to 30), and is further desirable in the range of M = 2N to 3N (in the case of N = 6, M = 12 to 18).

Then, in the embodiment, the restriction portion 17 provided in the first member 14 is designed to contact the bottom face of the locking mechanism fixing portion 11 during the assembly of the drug injection apparatus 1. Accordingly, it is possible to prevent the locking mechanism 5 from being separated from the drug discharge portion 6 compared to a case where the user pushes the drug discharge portion 6 into the fitting portion 18 at a strong pushing force. Hereinafter, a principle in which the locking mechanism 5 is separated from the drug discharge portion 6 and a function of the restriction portion 17 of the embodiment will be described.

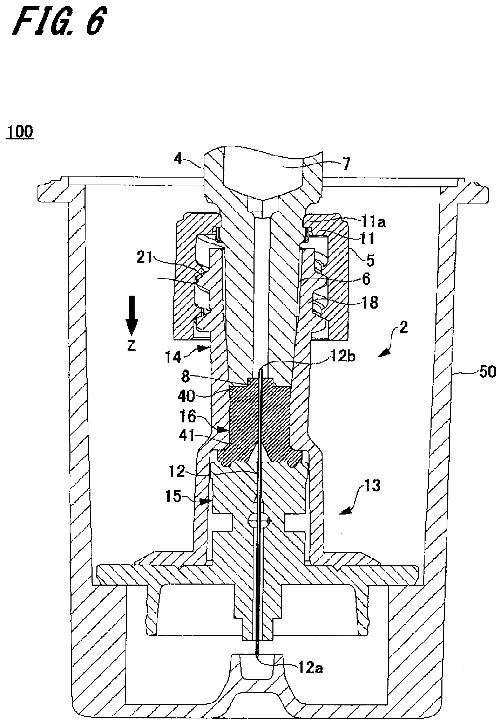

Fig. 6 is a schematic cross-sectional view of a drug injection apparatus 100 according to a comparative example. The drug injection apparatus 100 of the comparative example illustrated in Fig. 6 is different from the drug injection apparatus 1 of the embodiment only in that the restrictionportion is not provided. Thus, in Fig. 6, the same reference numerals will be given to the same components as those of Fig. 2 and a repetitive description thereof will be omitted. The drug injection apparatus 100 of the comparative example is described as a cross-sectional view of the drug injection apparatus 100 having a possibility that the locking mechanism 5 may be separated from the drug discharge portion 6 when the user pushes the drug discharge portion 6 into the fitting portion 18 at a strong pushing force.

Since the drug injection apparatus 100 according to the comparative example is not provided with the restrictionportion, a gap is formed between the end of the fitting hole 18a and the bottom face of the locking mechanism fixing portion 11 when the drug discharge portion 6 is fitted to the fitting portion 18 of the injection needle assembly 2 to be assembled thereto. In the drug injection apparatus 100 in such a state, when the user strongly and quickly assembles the syringe to the injection needle assembly 2, stress concentrates on the fitting claw 11a of the locking mechanism 5 fixing the locking mechanism 5 to the drug discharge portion 6 by engagement so that the fitting claw is broken. As a result, the lockingmechanism 5 is separated from the drug discharge portion 6. Here, the inventors analyze a reason of the breakage of the fitting claw 11a of the locking mechanism 5 by the following experiment.

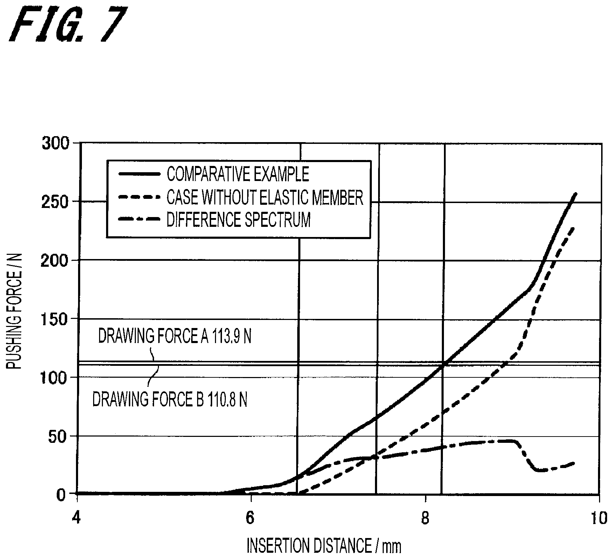

Fig. 7 is a diagram illustrating a measurement result of an insertion distance of the drug discharge portion 6 with respect to the fitting portion 18 and a pushing force generated by the threading of the male screw portion 21 into the female screw portion 23 after the drug discharge portion 6 is pushed into the fitting portion 18 and the injection needle assembly 2 is rotated relative to the drug discharge portion 6 in the drug injection apparatus 100 according to the comparative example. In Fig. 7, a horizontal axis indicates the insertion distance and a vertical axis indicates the pushing force. A measurement result of the drug injection apparatus 100 of the comparative example is indicated by a solid line and a measurement result obtained by a measurement in which the elastic member 16 is drawn out from the drug injection apparatus 100 of the comparative example is indicated by a dashed line. Further, such a difference (a difference spectrum) is illustrated as a result.

Further, Fig. 7 illustrates a drawing force at which the locking mechanism 5 is separated from the drug discharge portion 6 due to the breakage of the fitting claw 11a when the locking mechanism 5 is drawn out in a direction (that is, a direction toward the injection needle assembly 2) indicated by an arrow Z of Fig. 6 in the drug injection apparatus 100 of the comparative example. Additionally, the fitting claw 11a is broken when a drawing force exceeds a fitting force of the drug discharge portion 6 due to the fitting claw 11a of the locking mechanism 5.

Fig. 7 illustrates a drawing force A at which the locking mechanism 5 is drawn out in a direction parallel to the axial direction of the drug discharge portion 6 and a drawing force at which the lockingmechanism is drawn out in a direction inclined by 3° with respect to the axial direction of the drug discharge portion 6. A drawing force B at a limit in which the fitting claw 11a was broken when the locking mechanism 5 was drawn out in a direction parallel to the axial direction of the drug discharge portion 6 was 113.9 N and a drawing force at a limit in which the fitting claw 11a was broken when the locking mechanism 5 was obliquely drawn out in a direction inclined by 3° with respect to the axial direction of the drug discharge portion 6 was 110.8 N. From the results of the drawing force A and B, it is understood that a drawing force at a limit in which the fitting claw 11a is broken further decreases (that is, the fitting claw is easily broken by a small force) compared to a case where a force is obliquely applied to the locking mechanism 5 so that the locking mechanism is drawn out in a direction parallel to the axial direction of the drug discharge portion 6.

A relation between the insertion distance and the pushing force of the drug injection apparatus 100 with the elastic member 16 is likely to be substantially the same as a relation between the insertion distance and the pushing force of the drug injection apparatus 100 without the elastic member 16. Then, since a difference between a measurement result of the drug injection apparatus 100 of the comparative example and a measurement result obtained when the elastic member 16 was drawn out from the drug injection apparatus 100 of the comparative example was substantially uniform, it was proved that a repellent force of the elastic member 16 did not separate the locking mechanism 5 from the drug discharge portion 6.

As illustrated in Fig. 7, when an insertion distance of the drug discharge portion 6 with respect to the fitting portion 18 increases, a pushing force of the drug discharge portion 6 with respect to the fitting portion 18 abruptly increases. From Fig. 7, it is understood that the fitting claw 11a of the locking mechanism 5 is broken when a pushing force becomes equal to or larger than a drawing strength of a limit at which the fitting claw 11a of the locking mechanism 5 is broken. Thus, in order to prevent the breakage of the fitting claw 11a of the locking mechanism 5, it is found that a force larger than a drawing force at a limit in which the fitting claw 11a is broken does not need to be applied to the fitting claw 11a of the locking mechanism 5 when the drug discharge portion 6 is inserted into the fitting portion 18.

Since the drug injection apparatus 1 of the embodiment is provided with the restriction portion 17, the restriction portion 17 contacts the bottom face of the locking mechanism fixing portion 11 when the drug discharge portion 6 is inserted into the fitting portion 18 to a certain degree. Then, a force which is directed from the restriction portion 17 toward the bottom face of the locking mechanism fixing portion 11 is generated at a contact face between the bottom face of the locking mechanism fixing portion 11 and the restriction portion 17 and a force of pushing the locking mechanism 5 toward the injection needle assembly 2 is canceled at the contact face (the bottom face). Accordingly, even when the user quickly connects the syringe 3 and the injection needle assembly 2 to each other at a force stronger than a general force, a force of pushing the locking mechanism 5 toward the injection needle assembly 2 is restricted by the restriction portion 17. For this reason, it is possible to prevent a breakage of the fitting claw 11a of the locking mechanism 5. Accordingly, since it is possible to prevent the drug discharge portion 6 from being separated from the locking mechanism 5 when the user connects the syringe 3 and the injection needle assembly 2 to each other, it is possible to reliably connect the syringe 3 and the injection needle assembly 2 to each other.

Incidentally, the drug injection apparatus 100 according to the comparative example has a configuration in which the taper ratio of the fitting portion 18 is larger than the taper ratio of the drug discharge portion 6 as in the embodiment. In this way, in a case where the taper ratio of the fitting portion 18 is larger than the taper ratio of the drug discharge portion 6, there is a tendency that a pushing force rapidly increases with respect to an insertion distance of the drug discharge portion 6 compared to a case where the taper ratio of the drug discharge portion 6 is the same as the taper ratio of the fitting portion 18. That is, as in the embodiment, in the drug injection apparatus 1 in which the taper ratio of the fitting portion 18 is larger than the taper ratio of the drug discharge portion 6, a large force is abruptly applied to the fitting claw 11a of the lockingmechanism 5 compared to a case of the drug injection apparatus in which the taper ratio of the drug discharge portion 6 is the same as the taper ratio of the fitting portion 18. As a result, there is a high possibility of the breakage of the fitting claw 11a of the locking mechanism 5.

Thus, a configuration in which the restriction portion 17 is provided to restrict a pushing force applied to the fitting claw 11a of the locking mechanism 5 is particularly effective in a case where the taper ratio of the fitting wall 18c is larger than the taper ratio of the drug discharge portion 6 as in the drug injection apparatus 1 of the embodiment.

Further, it is desirable to set a length of the restriction portion 17 in the axial direction to a degree in which a pushing force larger than the drawing force indicated by Fig. 7 is not applied to the fitting claw 11a of the locking mechanism 5. That is, an insertion distance of the drug discharge portion 6 with respect to the fitting portion 18 is determined so that a pushing force becomes smaller than a drawing force at a limit in which the fitting claw 11a of the locking mechanism 5 is broken and a length of the restriction portion 17 in the axial direction is adjusted so that an insertion distance of the drug discharge portion 6 with respect to the fitting portion 18 does not exceed the determined value (that is, the drug discharge portion is not inserted anymore). Further, when the axial length of the restriction portion 17 is too short, the same problem as that of the comparative example occurs. However, when the axial length of the restriction portion is too long, an insertion distance of the drug discharge portion 6 with respect to the fitting portion 18 is shortened and thus a problem arises in that the fitting between the drug discharge portion 6 and the fitting portion 18 is weakened and a pressure resistance during the administration of the drug cannot be maintained. Thus, the axial length of the restriction portion 17 is adjusted so that an ideal insertion distance of the drug discharge portion 6 with respect to the fitting portion 18 is maintained. Since such a restriction portion 17 restricts an insertion distance of the drug discharge portion 6 with respect to the fitting portion 18 and also restricts a pushing force applied to the locking mechanism 5, a breakage of the fitting claw 11a of the locking mechanism 5 can be prevented.

The restriction portion 17 of the embodiment is formed as a cylindrical member, but, for example, a plurality of protrusion portions provided at the end face of the fitting hole 18a may be used as a restriction portion. In this case, for example, a plurality of protrusion portions which are formed uprightly from the end face of the fitting hole 18a in a direction parallel to the axial direction of the fitting portion 18 are provided at the same interval in the end face of the fitting hole 18a. Even in such a configuration, the same effect as that of the embodiment can be obtained.

Further, in the embodiment, since the restriction portion 17 is provided in the end face of the fitting hole 18a and the restriction portion 17 contacts the bottom face of the locking mechanism fixing portion 11 when the syringe 3 and the injection needle assembly 2 are connected to each other, a breakage of the fitting claw 11a of the locking mechanism 5 is prevented. However, the configuration of the restriction portion 17 is not limited thereto and a configuration may be employed which cancels a pushing force applied to the fitting claw 11a of the locking mechanism 5 when the syringe 3 and the injection needle assembly 2 are connected to each other. Hereinafter, a configuration of a drug injection apparatus with a restriction portion different from that of the embodiment will be described.

### «2. Second Embodiment»

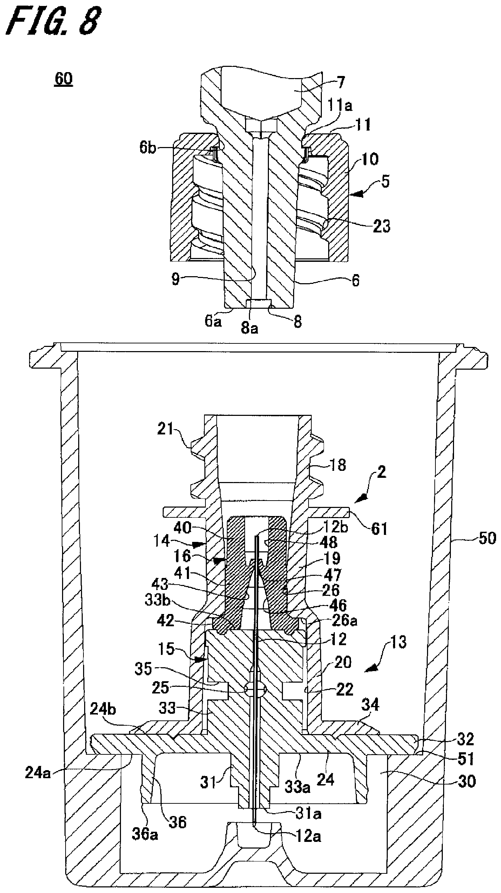

Fig. 8 is a schematic cross-sectional configuration diagram of a drug injection apparatus 60 according to a second embodiment of the invention. The drug injection apparatus 60 of the embodiment is different from the drug injection apparatus 1 according to the first embodiment in that a configuration of a restriction portion 61 is different. In Fig. 8, the same reference numerals will be given to the same components as those of Fig. 2 and a repetitive description thereof will be omitted.

The restriction portion 61 of the embodiment is formed as an annular flange which is provided at a position located near the insertion portion 20 farther than an area provided with the male screw portion 21 and corresponding to the outer peripheral face of the fitting portion 18 of the first member 14 and protrudes outward in the radial direction of the fitting portion 18. An outer diameter of the restriction portion 61 is formed to be substantially equal to or larger than an outer diameter of the locking mechanism 5. Then, the restriction portion 61 is provided at a position where the end face of the locking body 10 contacts a face of the restriction portion 61 near the locking mechanism 5 when the syringe 3 and the injection needle assembly 2 are connected to each other to be assembled. Regarding a position of the restriction portion 61, it is desirable to set the position to a degree in which an insertion distance of the drug discharge portion 6 with respect to the fitting portion 18 is maintained as an ideal insertion distance and a pushing force larger than the drawing force indicated by Fig. 7 is not applied to the fitting claw 11a of the locking mechanism 5 similarly to the first embodiment.

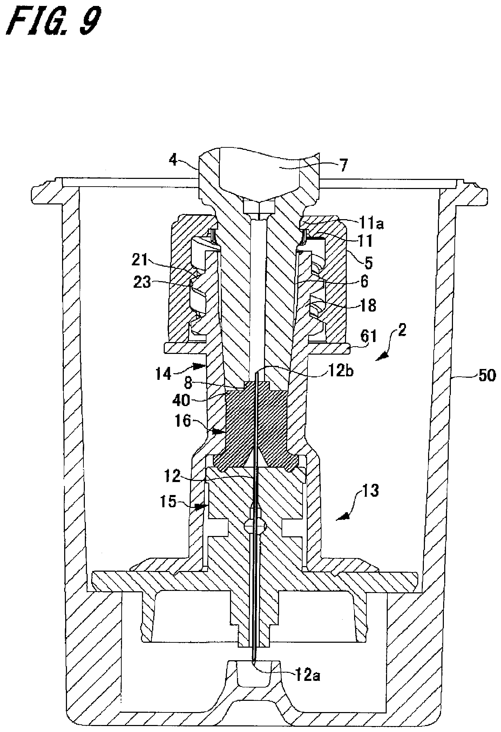

Fig. 9 is a schematic cross-sectional configuration diagram illustrating a case where the drug injection apparatus 60 of the embodiment is assembled. Even in the embodiment, when the syringe 3 and the injection needle assembly 2 are connected to each other, the drug discharge portion 6 is inserted into the fitting portion 18 to a certain degree so that the restriction portion 61 contacts the end face of the locking body 10. Then, a force which is directed from the restriction portion 61 toward the locking body 10 is generated in the contact face between the bottom face of the locking body 10 and the restriction portion 61 and a force of pushing the locking mechanism 5 toward the injection needle assembly 2 is canceled at the contact face (the bottom face). Accordingly, since a force of pushing the locking mechanism 5 toward the injection needle assembly 2 is restricted by the restriction portion 61 even when the user strongly and quickly connects the syringe 3 and the injection needle assembly 2 to each other, a breakage of the fitting claw 11a of the locking mechanism 5 can be prevented. Accordingly, since it is possible to prevent the drug discharge portion 6 from being separated from the locking mechanism 5 when the user connects the syringe 3 and the injection needle assembly 2 to each other, it is possible to reliably connect the syringe 3 and the injection needle assembly 2 to each other. Additionally, even in the embodiment, the same effect as that of the first embodiment can be obtained.

While certain embodiments of the drug injection apparatus and the injection needle assembly of the invention have been described, the invention is not limited to the above-described embodiments. Various modifications can be made without departing from the spirit of the invention.

For example, in the first and second embodiments, the taper ratio of the drug discharge portion 6 is set to N = 6, but may be appropriately changed. Additionally, in the first and second embodiments, the taper ratio of the drug discharge portion 6 is set to be different from the taper ratio of the fitting portion 18, but even when both taper ratios are the same as each other, the effect of the invention can be obtained.

Further, in the first and second embodiments, an example of the drug injection apparatus including the syringe 3 and the injection needle assembly 2 has been described, but the invention can be applied to a medical instrument in which a target connection portion such as a drug tube is connected to the drug discharge portion 6 including the locking mechanism 5. In this case, since the target connection portion is provided with the restriction portion 17 or 61 contacting the locking mechanism 5 provided in the connection portion, the same effect as that of the invention can be obtained.

### Reference Signs List

1, 60 ... drug injection apparatus, 2 ... injection needle assembly, 3 ... syringe, 4 ... syringe body, 5 ... locking mechanism, 6 ... drug discharge portion, 7 ... drug storage portion, 8 ... extension portion, 10 ... locking body, 11...locking mechanism fixing portion, 11a ... fitting claw, 12 ... hollow needle, 13 ... needle hub, 14 ... first member, 15 ... second member, 16 ... elastic member, 17, 61 ... restriction portion, 18 ... fitting portion, 18c ... fitting wall, 18d... guide wall, 18e ... connection wall, 19 ... intermediate portion, 20 ... insertion portion, 21 ... male screw portion, 23 ... female screw portion, 24 ... connection piece, 26 ... cylinder hole, 30 ... skin contact portion, 31... adj ustment portion, 32 ... guide portion, 33 ... base portion, 34 ... fixing piece, 35 ... injection hole, 36 ... stabilization portion, 40 ... deformation portion, 41 ... body portion, 42 ... flange portion, 43 ... insertion hole, 46 ... end face side separation portion, 47 ... adhesion portion, 48 ... contact face side separation portion, 50 ... cap

## Claims

1. An injection needle assembly configured for connection to a drug container including a cylindrical drug discharge portion having a male tapered shape with an outer diameter that decreases toward a distal end, and a locking mechanism having a first screw portion and being configured such that rotation of the locking mechanism relative to the drug discharge portion is restricted, the injection needle assembly comprising:
a hollow needle having a needle tip for puncturing skin;
a holding portion that holds the hollow needle;
a fitting portion which having a female tapered shape such that the drug discharge portion is inserted into the fitting portion, the fitting portion including a second screw portion on its outer peripheral face, the second screw portion being configured to be threaded into the first screw portion; and
a restriction portion configured to restrict a distance by which the drug discharge portion is pushed into the fitting portion by contacting the locking mechanism when the drug discharge portion is inserted into the fitting portion and the first screw portion is threaded into the second screw portion.

2. The injection needle assembly according to claim 1, wherein
the restriction portion is located at an end of a fitting hole of the fitting portion.

3. The injection needle assembly according to claim 1, wherein
the restriction portion is located at the outer peripheral face of the fitting portion.

4. The injection needle assembly according to any one of claims 1 to 3, wherein
when a taper ratio of the male tapered shape of the drug discharge portion is N/100, and a taper ratio of the female tapered shape of the fitting portion is M/100, a relation of M > N is established.

5. A drug injection apparatus comprising:
a syringe comprising a cylindrical drug discharge portion having a male tapered shape with an outer diameter that decreases toward a distal end and a locking mechanism having a first screw portion and being configured such that rotation of the locking mechanism relative to the drug discharge portion is restricted; and
an injection needle assembly configured to be connected to the syringe, the injection needle assembly comprising a hollow needle, a holding portion that holds the hollow needle, a fitting portion having a female tapered shape configured such that the drug discharge portion is inserted into the fitting portion, the fitting portion including a second screw portion on its outer peripheral face, the second screw portion being configured to be threaded into the first screw portion, and a restriction portion configured to restrict a distance by which the drug discharge portion is pushed into the fitting portion by contacting the locking mechanism when the drug discharge portion is inserted into the fitting portion and the first screw portion is threaded into the second screw portion.

6. The drug injection apparatus according to claim 5, wherein
the locking mechanism includes a cylindrical locking mechanism body, the first screw portion is located at an inner peripheral face of the cylindrical locking mechanism body, the locking mechanism further includes a fitting claw is fitted to the drug discharge portion,
the restriction portion is provided at an end of a fitting hole of the fitting portion, and
when the first screw portion is threaded into the second screw portion, the restriction portion contacts the locking mechanism at the a location adjacent to the fitting claw.

7. The drug injection apparatus according to claim 5, wherein
the locking mechanism includes a cylindrical locking mechanism body, the first screw portion is located at an inner peripheral face of the cylindrical locking mechanism body, the locking mechanism further includes a fitting claw that is fitted to the drug discharge portion,
the restrictionportion is providedat the outer peripheral face of the fitting portion, and
when the first screw portion is threaded into the second screw portion, the restriction portion contacts an end of the locking mechanism body.

8. A medical instrument for connection to a drug container including a cylindrical drug discharge portion having a male tapered shape with an outer diameter that decreases toward a distal end, and a locking mechanism having a first screw portion and being configured such that rotation of the locking mechanism relative to the drug discharge portion is restricted, the medical instrument comprising:
a fitting portion having a female tapered shape that the drug discharge portion is inserted into the fitting portion, the fitting portion including a second screw portion on its outer peripheral face, the second screw portion being configured to be threaded into the first screw portion; and
a restriction portion configured to restrict a distance by which the drug discharge portion is pushed into the fitting portion by contacting the locking mechanism when the drug discharge portion is inserted into the fitting portion and the first screw portion is threaded into the second screw portion.
